# EUROPÄISCHE PATENTSCHRIFT

(11) **EP 0 425 625 B1**
(45) Veröffentlichungstag und Bekanntmachung des Hinweises auf die Patenterteilung: **04.08.1993**
(21) Anmeldenummer: 90907010.4
(22) Anmeldetag: 09.05.1990
(51) Int. Cl.: A61F 5/04

(54) **FINGERSCHIENE**
FINGER SPLINT
ECLISSE POUR DOIGTS

(30) Priorität: 19.05.1989 DE 8906213 U; 13.12.1989 DE 8914648 U
(43) Veröffentlichungstag der Anmeldung: 08.05.1991
(73) Patentinhaber: Waldemar Link (GmbH & Co.), D-22315 Hamburg (DE)
(72) Erfinder: LINK, Helmut, D., D-2000 Hamburg 65 (DE)
(74) Vertreter: Glawe, Delfs, Moll & Partner
(86) Internationale Anmeldenummer: EP9000743
(87) Internationale Veröffentlichungsnummer: WO9014057

(56) Entgegenhaltungen:
- EP-A- 0 162 692
- EP-A- 0 183 021
- DE-A- 3 026 839
- DE-A- 3 517 073
- DE-U- 8 914 648
- US-A- 2 528 456

## Beschreibung

Die Erfindung bezieht sich auf eine Fingerschiene zur Fixierung des äußersten Fingergelenks in Streckstellung, bestehend aus einer das äußerste Fingergelenk und das Endglied unterseitig stützenden Schale und einer das äußerste Fingergelenk und das Mittelglied oberseitig stützenden Schale, die nahe dem äußersten Fingergelenk durch einen Ringteil miteinander verbunden sind.

Bei bekannten Fingerschienen dieser Art (DE-A 35 17 073, EP-A 0 183 021), sind die beiden Schalen in sich jeweils ungeteilt ausgeführt. Selbst wenn sie aus verformbarem Werkstoff ausgebildet sind, vermögen sie daher dennoch praktisch nicht nachzugeben. Das hat den Nachteil, daß die Schiene sich z.B. abklingenden Schwellungen an der Basis des Fingerendglieds nicht nachgiebig folgen kann. Bei den üblichen Anwendungsfällen von Fingerschienen, beispielsweise Endgliedsfrakturen, operativer Versorgung von Strecksehnenrupturen und Entzündungen des Nagelbereichs ist das Endglied des Fingers deutlich verdickt und empfindlich, besonders von dorsal. Dies zwingt zur Verwendung entsprechend größerer Schienen, die sich dann aber im Bereich des Fingermittelglieds als zu weit erweisen und deshalb schlecht passen und nicht richtig fixiert werden können. Sie verdrehen sich leicht und stützen nicht korrekt.

Gemäß der US-A 2 528 456 wird versucht, einer Fingerschiene in den unterschiedlichen Bereichen ihrer Längserstreckung eine Anpassung an unterschiedliche Fingerdurchmesser dadurch zu ermöglichen, daß sie zusammengesetzt wird aus einer Vielzahl von in Umfangsrichtung verlaufenden Zungen, die an einem gemeinsamen, durchgehenden Schienenteil befestigt sind. Die Zungen sind elastisch federnd ausgebildet, so daß sie sich der Dicke des jeweils von ihnen umfaßten Bereichs des Fingers anpassen können, und überlappen mit ihren Enden den durchgehenden Schienenteil. Die federnden Zungen haben den Nachteil, daß sie ständig und unvermeidlich Druck auf den Finger ausüben. Dies soll gerade durch denjenigen Typ von Fingerschienen vermieden werden, auf den sich die Erfindung bezieht, nämlich dadurch, daß zwei auf gegenüberliegenden Seiten des Fingers in unterschiedlichen Bereichen desselben anliegende, der Fingerform angepaßte, starr miteinander verbundene Schalen verwendet werden.

Aus der EP-A 0 162 692 ist eine Fingerschiene bekannt, die einen federnden Ringteil und davon ausgehende, sich in Längsrichtung erstreckende Zungen umfaßt, die durch verschiebbare Ringe zusammengehalten werden. Je nach Dicke des Fingers können engere oder weitere Ringe aufgeschoben werden, durch welche die in Längsrichtung verlaufenden Zungen enger oder weiter zusammengehalten werden. Dabei entspricht der eingestellte Abstand der Längszungen jeweils der dicksten Stelle des Fingers, während sie sich an die dünneren Bereiche nicht anlegen kann. Das hat zur Folge, daß in den dünneren Bereichen des Fingers keine korrekte Führung erreichbar ist.

Die DE-A 30 26 839 zeigt eine Fingerschiene derart, auf die sich auch die Erfindung bezieht. Sie ist durch ein Gelenk mit einem weiteren Schienenteil verbunden, der sich am Fingergrundglied abstützt und geschlitzt ist. Im Gegensatz dazu sind diejenigen Schienenteile, die das Endglied unterseitig und das Mittelglied oberseitig stützen, in der herkömmlichen Weise starr und ungeteilt ausgeführt. Diese Schrift legt daher Zeugnis ab für die bisherige medizinische Auffassung, daß die gattungsgemäße Art von Fingerschienen in sich starr sein muß, um die gewünschte, anatomisch angepaßte Stützwirkung erzielen zu können.

Der Erfindung liegt die Aufgabe zugrunde, eine Schiene der eingangs genannten Art zu schaffen, die die dort angegebenen Nachteile nicht aufweist.

Die erfindungsgemäße Lösung besteht darin, daß die oberseitige Schale geteilt ausgeführt ist und die Schiene aus elastisch oder plastisch nachgiebigem Material besteht.

Die Erfindung hält an der anatomischen Anpassung der Schienenform fest, vollzieht aber einen Bruch mit der Schulmeinung, daß sämtliche Teile dieser Schiene starr miteinander und in sich verbunden sein müßten, um die anatomisch richtige Stützwirkung erzielen zu können, allerdings wird dieser Bruch nur selektiv vollzogen, nämlich in Bezug auf die oberseitige Schale, welche das Mittelglied stützt. Unangetastet bleibt die Formsicherheit der Schale, die das Endglied und das äußerste Fingergelenk unterseitig stützt. Es hat sich gezeigt, daß auf diese Weise trotz der anatomischen Anpassung der Schienenform an die Fingerform und unter Beibehaltung der anatomisch richtigen Stützwirkung eine Anpassung im hinteren Schienenbereich möglich ist, die in den erwähnten Fällen, in denen durch Entzündung oder Bluterguß ein Teil des Fingers verdickt ist, zu einer sachgerechten Versorgung führen kann. Dies wird dadurch erreicht, daß der aus elastisch oder plastisch nachgiebigem Material bestehenden Schiene die Fähigkeit verliehen wird, entsprechend nachzugeben. Zweckmäßigerweise ist die Teilung durchgehend ausgebildet. Jedoch soll nicht die Möglichkeit ausgeschlossen sein, daß die getrennten Schalenteile im hinteren oder vorderen Bereich miteinander verbunden bleiben, was zur Stabilisierung der Schiene beiträgt, ohne ihre Dehnbarkeit insbesondere im Bereich des Fingerendglieds zu verringern.

Eine vorteilhafte Ausführung der Erfindung zeichnet sich dadurch aus, daß der die Mittelgliedstütze teilende Schlitz im entspannten Zustand der Fingerschiene nach hinten keilförmig breiter wird. Dies hat den Vorteil, daß die Mittelgliedstütze leicht zur Anpassung an das Mittelglied bzw. an das mittlere Fingergelenk zusammengebogen werden kann, wenn dies erforderlich ist, ohne daß dadurch die Gefahr einer gegenseitigen Überlappung der Schienenteile entsteht. Im Bereich des Ringteils besteht trotz der dort geringeren Breite des Schlitzes eine solche Gefahr im allgemeinen nicht, weil der Ringteil größere Steifigkeit besitzt als die nach hinten ragenden Teile der Mittelgliedstütze. Deshalb kann nach der Erfindung auch der Ringteil durch den Schlitz geteilt sein. Bei einer anderen vorteilhaften Ausführungsform ist jedoch der Ringteil ungeteilt, so daß der Schlitz oder die Schlitze lediglich im Bereich der Mittelgliedstütze mehrere im Ringteil untereinander verbundene Zungen voneinander abteilen. Letzteres hat den Vorteil, daß die Zusammenrückung der die Mittelgliedstütze bildenden Zungen die Weite der Schiene im Bereich des äußersten Fingergelenks nicht beeinflußt, was beispielsweise im Fall einer Schwellung dieses Gelenks wertvoll ist.

Die die Mittelgliedstütze bildenden Zungen haben zweckmäßigerweise eine etwa konstante Breite; jedoch ist es auch denkbar, daß sie nach hinten zu etwas schmaler werden. Der Abstand benachbarter Zungen im entspannten Zustand der Fingerschiene ist zumindest an ihrem hinteren Ende in der Größenordnung von etwa halber Zungenbreite oder gar mehr.

Bewährt hat sich beispielsweise eine Fingerschiene, deren Mittelgliedstütze von drei bis fünf etwa gleich breiten Zungen gebildet ist, die gemeinsam etwas mehr als die obere Hälfte des Fingermittelglieds umschließen.

Die Erfindung wird im folgenden näher unter Bezugnahme auf die Zeichnung erläutert, die ein vorteilhaftes Ausführungsbeispiel veranschaulicht. Darin zeigen:

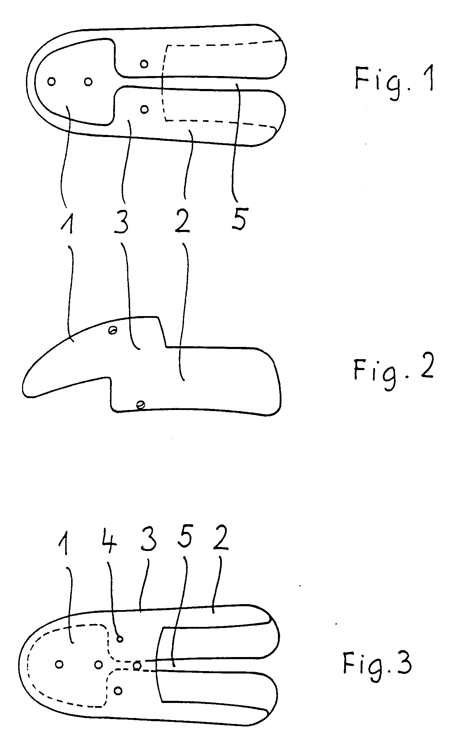

- Fig. 1: eine Oberansicht,
- Fig. 2: eine Seitenansicht und
- Fig. 3: eine Unteransicht einer ersten Ausführungsform der Fingerschiene und
- Fig. 4 bis 6: entsprechende Ansichten einer zweiten Ausführungsform

Die Fingerschiene besteht aus dem das Fingerendglied und das äußerste Fingergelenk unterseitig stützenden Schale 1, der das Fingermittelglied und das äußerste Fingergelenk oberseitig stützenden Schale 2, sowie dem die beiden Schalen auf beiden Seiten einstückig fest miteinander verbindenden, seitlichen Übergangsbereich 3, der sich mit dem hinteren Teil der Schale 1 und dem vorderen Teil der Schale 2 zu einem ringförmigen Abschnitt vereinigt. Die Schale 1 kann einen ovalen unterseitigen Ausschnitt aufweisen. Die Schale 2 kann einen größeren oberseitigen Ausschnitt aufweisen. Dies ist an sich bekannt. In der Zeichnung sind Ventilationsöffnungen 4 angedeutet.

Die Schale 2 ist mittig in Längsrichtung durch einen Schlitz 5 in zwei gleiche Schalenhälften geteilt. In der dargestellten Einstellung, in der die Fingerschiene ihre Normalweite aufweist, hat der Schlitz 5 eine Weite von 0 bis 5, vorzugsweise 1 bis 3 mm, so daß er nicht nur eine Aufweitung der Schiene sondern auch eine gewisse Umfangskontraktion erlaubt.

Im dargestellten Beispiel ist der Schlitz 5 gerade in Längsrichtung der Schiene ausgeführt. Jedoch kann er auch beispielsweise schräg, gebogen, gewellt oder gezahnt gestaltet sein.

In der zweiten Ausführungsform ist die Mittelgliedstütze 2 durch drei Schlitze 5 in vier Zungen 6 unterteilt, die ungefähr gleiche Breite haben. Da die Zungen 6 an ihrem vorderen Ende durch den Ringteil 3 fest miteinander verbunden sind, können sie sich dort auch dann nicht wesentlich einander nähern, wenn sie an ein vergleichsweise dünnes Fingermittelglied herangebogen werden. Sie können daher dort keinen unerwünschten Druck ausüben. Anders ist dies am hinteren Ende, wo die gegenseitige Näherung wesentlich größer sein kann. Deshalb sind die Schlitze 5 keilförmig nach hinten sich erweiternd ausgeführt.

Die Fingerschiene ist aus vergleichsweise leicht verformbarem Material wie Polyäthylen mit einer Dicke zwischen 1 und 1,5 Millimeter ausgeführt. Infolge ihrer gegenseitigen Trennung sind die den Stützteil 2 bildenden Zungen leicht verformbar. Trotzdem ergänzen sie einander überraschenderweise zu der erforderlichen Schienensteifigkeit, weil jedes denkbare Biegemoment, das im Bereich des Stützteils auf die Schiene übertragen werden könnte, in Richtung des größeren Widerstandsmoments zumindest einer der Zungen verläuft. Die Weichheit der Zungen um parallel zu ihrer dünnen Querschnittsrichtung verlaufende Biegeachsen machen den Stützteil daher anpassungsfähig, ohne seine für die Schienenfunktion notwendige Steifheit zu verringern. Wenn lediglich Biegemomente in bestimmten Vorzugsrichtungen aufzunehmen sind, ist es möglich, auf diejenigen Zungen zu verzichten, die in dieser Richtung keine wesentliche Steifigkeit erbringen. Wenn beispielsweise die Stütze lediglich gegenüber Biegemomenten zu wirken hat, die um zu den Gelenkachsen parallele Achsen wirken, kann beispielsweise auf die beiden mittleren Zungen verzichtet werden.

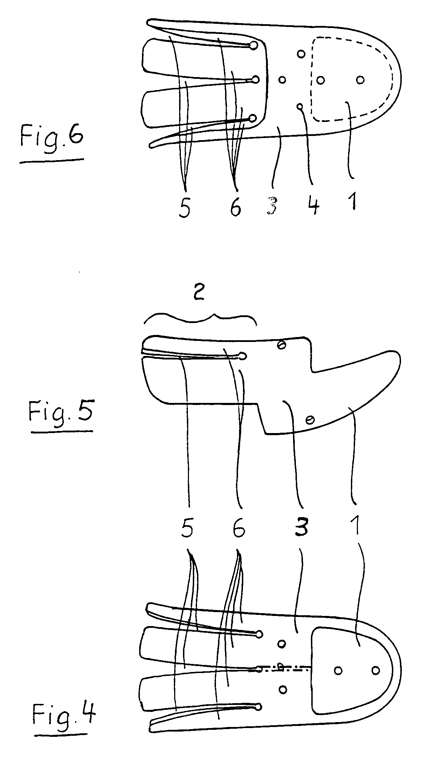

Strichpunktiert ist in Fig. 4 der Verlauf des mittleren Schlitzes 5 angedeutet, wenn dieser nicht nur die Mittelgliedstütze 2, sondern auch den Ringteil 3 durchtrennt.

## Patentansprüche

1. Fingerschiene zur Fixierung des äußersten Fingergelenks in Streckstellung, bestehend aus einer das äußerste Fingergelenk und das Endglied unterseitig stützenden Schale (1) und einer das äußerte Fingergelenk und das Mittelglied oberseitig stützenden Schale (2), die nahe dem äußersten Fingergelenk durch einen die Schalen (1,2) verbindenden Ringteil (3) einstückig miteinander verbunden sind, dadurch gekennzeichnet, daß die oberseitige Schale (2) geleilt ausgeführt ist (Längsschlitz 5) und die Schiene aus elastisch oder plastisch nachgiebigem Material besteht.

2. Fingerschiene nach Anspruch 1, dadurch gekennzeichnet, daß der Schlitz durchgehend ausgeführt ist.

3. Fingerschiene nach Anspruch 1 oder 2, dadurch gekennzeichnet, daß der die Mittelgliedstütze (2) teilende Schlitz (5) im entspannten Zustand der der Fingerschiene nach hinten keilförmig breiter wird.

4. Fingerschiene nach Anspruch 1 oder 3, dadurch gekennzeichnet, daß der Ringteil (3) ungeteilt ist.

5. Fingerschiene nach einem der Ansprüche 1 bis 4, dadurch gekennzeichnet, daß die Mittelgliedstütze (2) von mehreren im Ringteil (3) untereinander verbundenen Zungen (6) gebildet ist.

6. Fingerschiene nach Anspruch 5, dadurch gekennzeichnet, daß auch die die Zungen (6) voneinander trennenden Schlitze (5) im entspannten Zustand der Fingerschiene (1,2) nach hinten breiter werden.

7. Fingerschiene nach Anspruch 5 oder 6, dadurch gekennzeichnet, daß die Zungen (6) etwa konstante Breite haben.

8. Fingerschiene nach einem der Ansprüche 1 bis 7, dadurch gekennzeichnet, daß benachbarte Zungen (6) im entspannten Zustand der Fingerschiene (1,2) zumindest an ihrem hinteren Ende einen Abstand von etwa halber Zungenbreite oder mehr haben.

9. Fingerschiene nach einem der Ansprüche 1 bis 8, dadurch gekennzeichnet, daß die Mittelgliedstütze (2) von drei bis fünf etwa gleich breiten Zungen (6) gebildet ist.

## Claims

1. A finger splint for fixing the outermost finger joint in an extended position, comprising a cup (1) supporting the outermost finger joint and the end segment on the underside, and a cup (2) supporting the outermost finger joint and the medial segment on the upper side, which cups are joined together in one piece near the outermost finger joint by an annular part (3) joining the cups (1, 2), characterised in that the upper side cup (2) is divided (longitudinal slot 5) and the splint consists of resiliently or plastically flexible material.

2. A finger splint according to Claim 1, characterised in that the slot is continuous.

3. A finger splint according to Claim 1 or 2, characterised in that the slot (5) dividing the medial segment support (2) widens towards the rear in the shape of a wedge, in the unstressed condition of the finger split.

4. A finger splint according to Claim 1 or 3, characterised in that the annular part (3) is undivided.

5. A finger splint according to any one of Claims 1 to 4, characterised in that the medial segment support (2) is formed by a plurality of tongues (6) connected with one another in the annular part (3).

6. A finger splint according to Claim 5, characterised in that the slots (5) separating the tongues (6) from one another widen towards the rear, in the unstressed condition of the finger split.

7. A finger splint according to Claim 5 or 6, characterised in that the tongues (6) are of approximately constant width.

8. A finger splint according to any one of Claims 1 to 7, characterised in that, in the unstressed condition of the finger split (1, 2), adjacent tongues (6) have at least at their rear end a distance apart of approximately half the tongue width or more.

9. A finger splint according to any one of Claims 1 to 8, characterised in that the medial segment support (2) is formed by three to five tongues (6) of approximately equal width.

## Revendications

1. Attelle de doigt pour l'immobilisation de la dernière articulation du doigt en position d'extension, se composant d'une coquille (1) qui soutient par-dessous la dernière articulation du doigt et la phalangette, et d'une coquille (2) qui soutient par-dessus la dernière articulation du doigt et la phalangine, coquilles qui sont unies entre elles d'une seule pièce, à proximité de la dernière articulation du doigt, par une partie annulaire (3) reliant les coquilles (1, 2), caractérisée en ce que la coquille du dessus (2) est réalisée sous forme divisée (fente longitudinale 5), et en ce que l'attelle est faite d'une matière flexible élastiquement ou plastiquement.

2. Attelle de doigt selon la revendication 1, caractérisée en ce que la fente est formée de bout en bout.

3. Attelle de doigt selon la revendication 1 ou 2, caractérisée en ce qu'à l'état détendu de l'attelle de doigt, la fente (5) qui partage le soutien (2) de la phalangine s'élargit vers l'arrière en forme de coin.

4. Attelle de doigt selon la revendication 1 ou 3, caractérisée en ce que la partie annulaire (3) est non divisée.

5. Attelle de doigt selon l'une quelconque des revendications 1 à 4, caractérisée en ce que le soutien (2) de la phalangine est formé de plusieurs languettes (6) qui sont unies entre elles dans la partie annulaire (3).

6. Attelle de doigt selon la revendication 5, caractérisée en ce qu'à l'état détendu de l'attelle de doigt (1, 2), les fentes (5) qui séparent les languettes (6) les unes des autres s'élargissent également vers l'arrière.

7. Attelle de doigt selon la revendication 5 ou 6, caractérisée en ce que les languettes (6) ont une largeur à peu près constante.

8. Attelle de doigt selon l'une quelconque des revendications 1 à 7, caractérisée en ce qu'à l'état détendu de l'attelle de doigt (1, 2), les languettes (6) voisines sont séparées, à leur extrémité arrière, par une distance à peu près égale à une demi-largeur de languette ou plus.

9. Attelle de doigt selon l'une quelconque des revendications 1 à 8, caractérisée en ce que le soutien (2) de la phalangine est constitué par trois à cinq languettes (6) de largeur à peu près égale.
